(19) 

**Europäisches Patentamt**
**European Patent Office**
**Office européen des brevets**

(11) **EP 4 671 679 A1**

(12) **EUROPEAN PATENT APPLICATION**

(43) Date of publication:
**31.12.2025 Bulletin 2026/01**

(21) Application number: **25183920.5**

(22) Date of filing: **19.06.2025**

(51) International Patent Classification (IPC):
**G01B 11/25** (2006.01)

(52) Cooperative Patent Classification (CPC):
**G01B 11/2513; A61B 1/00194**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **28.06.2024 DK PA202470182**

(71) Applicant: 3Shape A/S
**1060 Copenhagen K (DK)**

(72) Inventors:
• **JONAS, Michael**
  **1060 Copenhagen K (DK)**
• **JUUL, Frederik**
  **1060 Copenhagen K (DK)**
• **CHRISTIANSEN, Mads Brøkner Falkenberg**
  **1060 Copenhagen K (DK)**

(74) Representative: **Guardian**
**IP Consulting I/S**
**Diplomvej, Building 381**
**2800 Kgs. Lyngby (DK)**

(54) **A SYSTEM AND METHOD FOR GENERATING THREE-DIMENSIONAL REPRESENTATION OF DENTAL OBJECT**

(57) The present disclosure relates to an intraoral scanning system (102) that is configured to generate a 3D representation of a dental object (128). The system receives signal information (132) from the plurality of sensor elements (114), wherein the signal information is associated with one or more events at the plurality of sensor elements. The system determines a sequence of events associated with one or more sensor elements from the plurality of sensor elements based on the received signal information, determines position information of the focus element (112) with respect to at least the part of the dental object based on the sequence of events, and generates at least one 3D point associated with at least the part of the dental object for the 3D representation of the dental object based on the position information.

FIG. 1A

EP 4 671 679 A1

## Description

### TECHNICAL FIELD

An example embodiment of the present disclosure generally relates to intraoral scanner and more particularly relates to an intraoral scanning system and a method for intraoral scanning for generating a three-dimensional representation of a dental object using event sensors.

### BACKGROUND OF THE INVENTION

Intraoral scanners are electronic devices that may be used for, for example, capturing digital images of an oral cavity of a subject. In an example, the intraoral scanners may include light sources that may project light rays onto an object to be scanned, such as teeth, gums, and other intraoral structures inside the oral cavity of the subject. In certain cases, computer-aided design process may be used to create a virtual three-dimensional (3D) model of the teeth of the subject using digital images captured by the intraoral scanner. The digital images of the teeth are imported into a computer-aided design (CAD) program, which creates a final virtual 3D model of dentition of the teeth of the subject.

Typically, intraoral scanners are used for examination or treatments inside oral cavities of subjects. The use of intraoral scanners may eliminate the use of conventional impression material and plaster models, simplify clinical treatment procedures of teeth for the dentists, as well as reduce user discomfort. However, to create accurate virtual 3D models of dentition of the teeth of subjects, the intraoral scanner may be required to capture highly accurate 3D images. Moreover, the intraoral scanner may leverage the use of field programmable gate arrays (FPGA) to allow for the fast and efficient processing of the images. In operation, the intraoral scanner may have to be moved over an oral cavity of a subject, thereby capturing a series of images at different depths. This may require significant memory allocation and computational power. Further, the FPGA may perform correlation calculations on the captured series of images to determine focal planes for each pixel for creating the 3D model of the oral cavity. In such a case the intraoral scanner must calculate focus for each pixel in the series of images. This may be a time consuming and resource intensive process that creates a significant bottleneck in the scanning process.

Therefore, there is a need for improved systems and methods of intraoral scanning to overcome the limitations associated with conventional methods for intraoral scanning to generate the 3D model of dental structures of the oral cavities.

### SUMMARY

An intraoral scanning system, a method and a computer programmable product are provided for generating a 3D representation of a dental object using a hand-held intraoral scanner. The hand-held intraoral scanner comprises a focus element to focus a light signal and a plurality of sensor elements to detect a reflected light signal.

It is an objective of the present disclosure to provide techniques for generating accurate 3D representation of the dental object while reducing memory and computation power required, thereby overcoming the limitations associated with conventional methods for intraoral scanning.

In one aspect, an intraoral scanning system configured to generate a 3D representation of a dental object is provided. The intraoral scanning system may include a hand-held intraoral scanner including at least one light sensor. The at least one light sensor may include a focus element to focus a light signal on at least a part of the dental object, and a plurality of sensor elements to detect a reflected light signal from at least the part of the dental object. The intraoral scanning system may further include one or more processors connected to the hand-held intraoral scanner. The one or more processors may be configured to receive signal information from the plurality of sensor elements. The signal information may be associated with one or more events at the plurality of sensor elements. The one or more processors may further be configured to determine a sequence of events associated with one or more sensor elements from the plurality of sensor elements based on the received signal information, determine position information of the focus element with respect to at least the part of the dental object based on the sequence of events, and generate at least one 3D point associated with at least the part of the dental object for the 3D representation of the dental object based on the position information.

An event of the sequence may include information about whether the light intensity detected by a sensor element of at least the light sensor corresponds to an up event or a down event. The event is an up event when the detected light intensity is above a first threshold value, and the event is a down event when the detected light intensity is below a second threshold value. This means, a detected light intensity that is between the first and the second threshold value would not generate an event.

In accordance with some example embodiments, the hand-held intraoral scanner further includes a projector unit to generate the light signal, a pattern generator to generate a plurality of configurations in form of an illumination pattern using the generated light signal, and an optical unit for transmitting the light signal towards the dental object along an optical path by illuminating at least the part of the dental object with the generated light signal. The optical unit is also configured to transmit the reflected light signal from the dental object to the at least one light sensor.

In accordance with some example embodiments, the illumination pattern is a checkerboard pattern.

Further, the one or more processors are configured to compare a first value of light intensity of the reflected light signal for at least one sensor element from the plurality of sensor elements with a first threshold value for the corresponding at least one sensor element and identify an event from the sequence of events associated with the at least one sensor element based on the comparison.

In accordance with some example embodiments, the one or more processors are further configured to identify a lack of event for the at least one sensor element in response to determining the first value to be equal to the first threshold value. The one or more processors are further configured to identify an up event for the at least one sensor element in response to determining the first value to be greater than the first threshold value. The one or more processors are further configured to identify a down event for the at least one sensor element in response to determining the first value to be less than the first threshold value or the less than a second threshold value that is lower than the first threshold value.

In accordance with some example embodiments, the one or more processors are further configured to determine a second threshold value and a third threshold value for the at least one sensor element based on the first value of light intensity in response to identifying an event from the sequence of events associated with the at least one sensor element. The one or more processors are further configured to determine a second value of light intensity associated with a second reflected light signal captured after the reflected signal, compare the second value with each of the second threshold value and the third threshold value for the at least one sensor element, and identify another event from the sequence of events associated with the at least one sensor element based on the comparison.

In accordance with some example embodiments, the one or more processors are further configured to control a movement of the focus element to project the illumination pattern. The movement of the focus element changes a depth associated with at least one of the plurality of sensor elements for capturing the reflected light signal.

In accordance with some example embodiments, the one or more processors are further configured to determine a frequency of the sequence of events, compare the frequency of the sequence of events with a threshold frequency, and determine the position information of the focus element with respect to at least the part of the dental object in response to determining the frequency to be greater than the threshold frequency.

In accordance with some example embodiments, the one or more processors are further configured to determine one or more sequences of events associated with the plurality of sensor elements based on the received signal information. The one or more processors are further configured to determine position information of the focus element with respect to one or more parts of the dental object based on each of the one or more sequences of events. The one or more processors are further configured to generate a 3D point cloud associated with the dental object for the 3D representation of the dental object based on the position information for each of the one or more sequences of events.

In accordance with some example embodiments, the at least one light sensor includes an event sensor configured to generate the signal information based on detecting a change in a light intensity of the reflected light signal to be greater than a threshold intensity, and an image sensor configured to capture image frames

In accordance with some example embodiments, the image sensor includes at least one of: a red, green, blue (RGB) sensor, an Infrared (IR) sensor, a Near IR sensor, or an ultraviolet (UV) sensor.

In accordance with some example embodiments, the one or more processors are configured to determine a first event of the sequence of events at one or more of the plurality of sensor elements using the event sensor. In an example, the determination of the first event triggers the image sensor to capture a first image with respect to at least the part of the dental object corresponding to the first event.

In accordance with some example embodiments, the one or more processors are further configured to generate the at least one 3D point associated with at least the part of the dental object based on the sequence of events detected by the event sensor, determine colour information associated with at least the part of the dental object based on at least the first image captured by the image sensor, and generate a 3D model of at least the part of the dental object based on the 3D point and the colour information .

In accordance with some example embodiments, each of the plurality of sensor elements is associated with one or more event sensors. In an example, each of the plurality of sensor elements operate asynchronously to capture the reflected light signal to identify an event from the sequence of events.

In accordance with some example embodiments, the signal information includes at least location information and timestamp information.

In accordance with some example embodiments, the one or more processors are further configured to determine a peak value of light intensity associated with the reflected light signal and generate the at least one 3D point associated with at least the part of the dental object based on the peak value.

In another aspect, a method for generating a 3D representation of a dental object is provided. The method is implemented using an intraoral scanning system comprising a hand-held intraoral scanner comprising at least one light sensor. The at least one light sensor includes a focus element to focus a light signal on at least a part of the dental object, and a plurality of sensor elements to detect a reflected light signal from at least the part of the dental object. The method comprises receiving signal information from the plurality of sensor elements, wherein the signal information is associated with one or more events at the plurality of sensor elements. The method further includes determining a sequence of events associated with one or more sensor elements from the plurality of sensor elements based on the received signal information. The method further includes determining position information of the focus element with respect to at least the part of the dental object based on the sequence of events. The method further includes generating at least one 3D point associated with at least the part of the dental object for the 3D representation of the dental object based on the position information.

In yet another aspect, a computer programmable product is provided. The computer programmable product comprises a non-transitory computer readable medium having stored thereon computer executable instructions, which when executed by a processing circuitry, cause the processing circuitry to carry out operations. The operations comprise receiving signal information from the plurality of sensor elements, wherein the signal information is associated with one or more events at the plurality of sensor elements. The operations further comprise determining a sequence of events associated with one or more sensor elements from the plurality of sensor elements based on the received signal information. The operations further comprise determining position information of the focus element with respect to at least the part of the dental object based on the sequence of events. The operations further comprise generating at least one 3D point associated with at least the part of the dental object for the 3D representation of the dental object based on the position information.

The foregoing summary is illustrative only and is not intended to be in any way limiting. In addition to the illustrative aspects, embodiments, and features described above, further aspects, embodiments, and features will become apparent by reference to the drawings and the following detailed description.

**EFFECT(S) OF THE INVENTION**

According to the present disclosure, an intraoral scanning system, a method and a computer programmable product are provided. One of the purposes of the present disclosure is to provide an accurate 3D representation of dental objects, thereby overcoming the limitations associated with conventional methods for intraoral scanning. Further, the conventional methods for performing intraoral scanning require significant processing power and memory allocation owing to requirement of processing of each pixel of a large number of images captured during scanning process. Therefore, the conventional scanning methods are computationally expensive, and power consuming. Further, the conventional methods include computational bottleneck created by the correlation calculations which can slow down the scanning process and increase the cost of processing the images captured by the intraoral scanner.

In the present disclosure, the hand-held intraoral scanner employs the focus element that may be movable (for example, a moving lens) to project an illuminating pattern (for example, but not limited to, a checkerboard pattern), onto a dental object at a plurality of focus depths. The movable focus element may facilitate to move the focus while taking one or more images of the dental object at a high speed. Further, the hand-held intraoral scanner captures the one or more images where the illuminating pattern is in focus on at least a part of the dental object. This may facilitate to identify a distance between the intraoral scanner and the part of the dental object. As a result, a position of the movable focus element may be known for each image from the one or more images. This may facilitate to determine which areas for each image from the one or more images are in focus. To this end, the images that are captured are based on identification of an event which further indicates that at least a part of the dental object is in focus. Such event-specific capturing of images substantially reduces the processing power required to process the images for generating 3D model of the dental object.

The disclosed intraoral scanner determines pixels (pixel groups) for each image from the one or more images that may be in focus to perform contrast correlation calculations on each pixel group for each captured image from the one or more images. Further, if a peak value associated with the reflected light is detected, then a 3D point associated with the dental object is generated. This may reduce significant processing power and memory allocation, thereby resulting in computationally inexpensive, and power saving method for the intraoral scanning. Further, the proposed method speeds up the scanning process by reducing the computational bottleneck.

**BRIEF DESCRIPTION OF THE DRAWINGS**

The present disclosure is illustrated by way of example and not by way of limitation in the figures of the accompanying drawings, in which the like reference numerals indicate like elements and in which:

FIG. 1A illustrates an exemplary network environment in which an intraoral scanning system for intraoral scanning is implemented, in accordance with an example embodiment;

FIG. 1B and FIG. 1C show example schematic diagrams of the intraoral scanning system 102 of FIG. 1A, in accordance with different example embodiments;

FIG. 2 illustrates a sequence diagram for generating a 3D representation of a dental object, in accordance with an example embodiment;

FIG. 3A illustrates a schematic diagram of an optical path followed by a light signal, in accordance with an example embodiment;

FIG. 3B illustrates an exemplary illuminating pattern, in accordance with an example embodiment;

FIG. 4A, FIG. 4B, and FIG. 4C illustrate exemplary graphical representations indicating a change in light intensity with respect to a focus position associated with a focus element, in accordance with various example embodiments;

FIG. 5 illustrates a flowchart of a method for generating a 3D representation of the dental object, in accordance with an example embodiment; and

FIG. 6 illustrates a block diagram of a hand-held intraoral scanner, in accordance with an example embodiment.

DETAILED DESCRIPTION

In the following description, for purposes of explanation, numerous specific details are set forth in order to provide a thorough understanding of the present disclosure. It will be apparent, however, to one skilled in the art that the present disclosure may be practiced without these specific details. In other instances, systems and methods are shown in block diagram form only in order to avoid obscuring the present disclosure.

Reference in this specification to "one embodiment" or "an embodiment" means that a particular feature, structure, or characteristic described in connection with the embodiment is included in at least one embodiment of the present disclosure. The appearance of the phrase "in one embodiment" in various places in the specification are not necessarily all referring to the same embodiment, nor are separate or alternative embodiments mutually exclusive of other embodiments. Further, the terms "a" and "an" herein do not denote a limitation of quantity, but rather denote the presence of at least one of the referenced items. Moreover, various features are described which may be exhibited by some embodiments and not by others. Similarly, various requirements are described which may be requirements for some embodiments but not for other embodiments.

Some embodiments of the present disclosure will now be described more fully hereinafter with reference to the accompanying drawings, in which some, but not all, embodiments of the disclosure are shown. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present disclosure. Further, the terms "processor", "controller" and "processing circuitry" and similar terms may be used interchangeably to refer to the processor capable of processing information in accordance with embodiments of the present disclosure. Further, the terms "electronic equipment", "electronic devices" and "devices" are used interchangeably to refer to electronic equipment monitored by the system in accordance with embodiments of the present disclosure. Thus, use of any such terms should not be taken to limit the spirit and scope of embodiments of the present disclosure.

The embodiments are described herein for illustrative purposes and are subject to many variations. It is understood that various omissions and substitutions of equivalents are contemplated as circumstances may suggest or render expedient but are intended to cover the application or implementation without departing from the spirit or the scope of the present disclosure. Further, it is to be understood that the phraseology and terminology employed herein are for the purpose of the description and should not be regarded as limiting. Any heading utilized within this description is for convenience only and has no legal or limiting effect.

As used in this specification and claims, the terms "for example" "for instance" and "such as", and the verbs "comprising," "having," "including" and their other verb forms, when used in conjunction with a listing of one or more components or other items, are each to be construed as open ended, meaning that that the listing is not to be considered as excluding other, additional components or items. Other terms are to be construed using their broadest reasonable meaning unless they are used in a context that requires a different interpretation.

An intraoral scanning system, a method and a computer programmable product are provided for generating a 3D representation of a dental object of a subject to provide an improved intraoral scanning process that is computationally inexpensive and power saving.

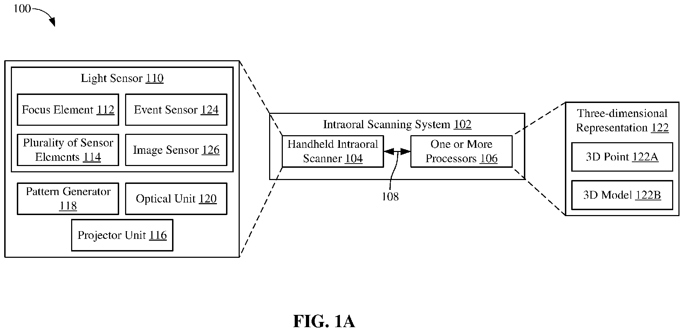

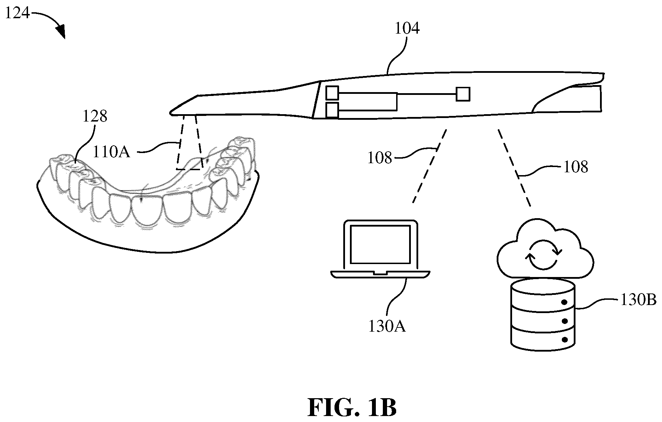

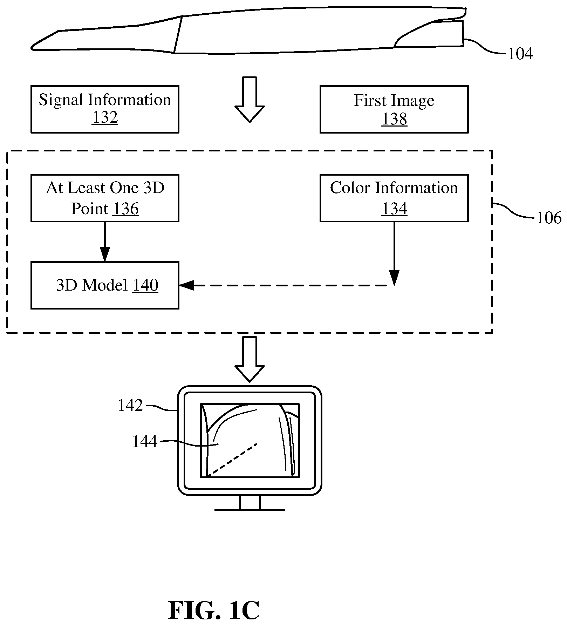

For instance, an exemplary network environment of the intraoral scanning system for intraoral scanning and generating the 3D representation of the dental object is provided below with reference to FIG. 1A, FIG. 1B, and FIG. 1C.

FIG. 1A illustrates an exemplary network environment 100 in which an intraoral scanning system 102 for intraoral scanning is implemented, in accordance with an example embodiment. The intraoral scanning system 102 may be used to generate a 3D representation of a dental object within oral cavity of a subject. For example, the intraoral scanning system 102 may be used by a user, such as a person having knowledge of dentistry, for example, a dentist, a dental technician, and so forth. Further, it is possible that one or more components of the network environment 100 and/or the intraoral scanning system 102 may be rearranged, changed, added, and/or removed without deviating from the scope of the present disclosure.

The intraoral scanning system 102 includes a hand-held intraoral scanner 104, and one or more processors 106. The network environment 100 may further include communication channels 108 that may be configured to establish communicative coupling between components of the intraoral scanning system 102, i.e., the hand-held intraoral scanner 104 and the one or more processors 106 (referred to as the processors 106, hereinafter).

For example, the processors 106 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processors 106 may include one or more processing cores configured to perform independently. A multi-core processor may enable multiprocessing within a single physical package. Additionally, or alternatively, the processors 106 may include one or more processors configured in tandem via the bus to enable independent execution of instructions, pipelining and/or multithreading. Additionally, or alternatively, the processors 106 may include one or more processors capable of processing large volumes of workloads and operations to provide support for big data analysis. In an example embodiment, the processors 106 may be in communication with the other components of the intraoral scanning system 102 (referred to as system 102, hereinafter) via a bus or the communication channel 108 for passing information among components of the system 102.

In an example, when the processors 106 are embodied as an executor of software instructions, the instructions may specifically configure the processors 106 to perform the algorithms and/or operations described herein when the instructions are executed. However, in some cases, the processors 106 may be a processor specific device (for example, a mobile terminal or a fixed computing device) configured to employ an embodiment of the present disclosure by further configuration of the processors 106 by instructions for performing the algorithms and/or operations described herein. The processors 106 may include, among other things, a clock, an arithmetic logic unit (ALU) and logic gates configured to support operation of the processors 106. The network environment, such as, 100 may be accessed using the communication channel 108.

The communication channel 108 may be wired, wireless, or any combination of wired and wireless communication networks, such as cellular, wireless fidelity (Wi-Fi), internet, local area networks, or the like. In accordance with an embodiment, the communication channel 108 may be one or more wireless full-duplex communication channels. In one embodiment, the communication channel 108 may include one or more networks such as a data network, a wireless network, a telephony network, or any combination thereof. It is contemplated that the data network may be any local area network (LAN), metropolitan area network (MAN), wide area network (WAN), a public data network (e.g., the Internet), short range wireless network, or any other suitable packet-switched network, such as a commercially owned, proprietary packet-switched network, e.g., a proprietary cable or fibre-optic network, and the like, or any combination thereof. In addition, the wireless network may be, for example, a cellular network and may employ various technologies including enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., worldwide interoperability for microwave access (WiMAX), Long Term Evolution (LTE) networks (for e.g. LTE-Advanced Pro), 5G New Radio networks, ITU-IMT 2020 networks, code division multiple access (CDMA), wideband code division multiple access (WCDMA), wireless fidelity (Wi-Fi), wireless LAN (WLAN), Bluetooth, Internet Protocol (IP) data casting, satellite, mobile ad-hoc network (MANET), and the like, or any combination thereof. The hand-held intraoral scanner 104 may be configured to communicate with the processors 106 via the communication channel 108.

The system 102 may be configured to store, such as in a memory, data generated by the system 102. For example, the system 102 may be configured to store data captured by the hand-held intraoral scanner 104, i.e., signal information. In an example, the signal information sensed, measured or determined by the hand-held intraoral scanner 104 includes a sequence of events associated with one or more sensor elements from a plurality of sensor elements 114. Moreover, the system 102 may be configured to store data generated by the processors 106. Such data may include a three-dimensional (3D) representation 122, i.e., a three-dimensional (3D) point 122A associated with at least a part of the dental object of the subject and a 3D model 122B at least the part of the dental object of the subject.

The intraoral scanning system 102 may be utilized for registration of intraoral scans and generating the 3D representation for the dental object to show surface information and/or inner layers of the dental object. The intraoral scanning system 102 may include multiple components, such as the hand-held intraoral scanner 104, the processors 106 and memory (not shown) that may communicate with each other to register the intraoral scans of the dental object.

In an example, the hand-held intraoral scanner 104 may include the processors 106. In another example, the hand-held intraoral scanner 104 may be coupled to the processors 106, wherein the processors 106 may be located remotely and may perform operations associated with the intraoral scanning system 102. The intraoral scanning system 102 may have enhanced processing capabilities that may be required to process the signal information and, in some cases, colour information in real time to generate the 3D model 122B of at least the part of the dental object.

The hand-held intraoral scanner 104 includes at least one light sensor 110. The at least one light sensor 110 may detect light signal and convert the detected light signal to an electrical signal for processing. This may facilitate to measure intensity of light signal. Examples of the light sensor 110 may include, but are not limited to, photoresistors, photodiodes, phototransistors and digital light sensors.

In an example, the light sensor 110 may include, but is not limited to, a focus element 112 to focus the light signal on at least a part of the dental object, and a plurality of sensor elements 114 to detect a reflected light signal from at least the part of the dental object. In an example, the focus element 112 ensures that the light signal is focused on at least the part of the dental object, thereby creating a sharp and clear image thereof. Example of the focus element 112 may include, but is not limited to, a focal lens or a group of lenses that may move to adjust the focus. The movement of the focus element 112 may modify a distance between the focus element 112 and the light sensor 110, thereby aligning an image plane with the light sensor 110 to create the clear image. In an example, the focus element 112 may move automatically to achieve sharp focus on at least the part of the dental object. In another example, the focus element 112 may be moved manually to achieve sharp focus on at least the part of the dental object.

Further, the plurality of sensor elements 114 may capture and provide signal information corresponding to at least the part of the dental object of the subject to the processors 106 based on the light signal sensed by the plurality of sensor elements 114. The plurality of sensor elements 114 may be designed to detect a change in the intensity of the light signal or an event. Thereafter, the plurality of sensor elements 114 may trigger a response based on the detection. The plurality of sensor elements 114 may capture the light signal and generate the signal information when the event is detected.

The handheld intraoral scanner 104 may include a projector unit 116 configured to generate or emit the light signal at different wavelengths, such as at near-infrared wavelength, infrared wavelength, white-coloured wavelengths and/or coloured visible wavelengths onto at least the dental object. In an example, the projector unit 116 may be configured to generate the light signal with different wavelengths onto at least the part of the dental object, wherein the different wavelengths include a near-infrared (NIR) wavelength, an infrared (IR) wavelength, ultraviolet (UV) wavelength, and a visible wavelength.

In an example, the light signal emitted by the projector unit 116 may include one or more colour lights of the filtered visible light signals, such as red, green, blue, and white. The projector unit 116 may include multiple light sources that are configured to emit the one or more colours light signals and the infrared light signal. The multiple light sources may be arranged within a single module that includes multiple Light Emitting Diodes (LEDs) that are configured to emit different wavelengths within the visible and non-visible wavelength ranges. In another example, light source, i.e., one or more LEDs that may be configured to emit infrared light signal, may be arranged separately from the light source that is configured to generate the visible light signal.

Examples of the projector unit 116 may include, but are not limited to, a structured light projector, a laser light projector, and a digital light processing projector. The structured light projector may generate a structured light to project an illumination pattern onto a surface of the dental object. The laser projector employs parallel confocal imaging with laser scanning to project precise laser patterns. The digital light processing projector may utilize digital light processing to project high-resolution patterns.

The handheld intraoral scanner 104 may include a pattern generator 118 configured to generate, using the generated light signal, a plurality of configurations in form of an illumination pattern. Specifically, the pattern generator 118 may correspond to a device or a software tool designed to generate a specific sequences or configurations of the light signal. In an example, the illumination pattern may include, but not limited to grid patterns, parallel strip patterns, or random or structured dot patterns. In an example, the illumination pattern is a checkerboard pattern. While in operation, the focus element 112 may be configured to direct or focus the generated illumination pattern onto at least the part of the dental object. This may facilitate accurate capturing of surface topology of at least the part of the dental object.

The handheld intraoral scanner 104 may include an optical unit 120. The optical unit 120 may correspond to an assembly of one or more optical components working together to capture detailed images of the dental object. The optical unit 120 may transmit the light signal towards the dental object along an optical path by illuminating at least the part of the dental object with the generated light signal. Further, the optical unit 120 may transmit the reflected light signal from the dental object to the at least one light sensor 110.

In an example, the at least one light sensor 110 may further include an event sensor 124 configured to generate the signal information. In an example, the event sensor 124 is configured to capture the signal information from the plurality of sensor elements 114. In an example, the processors 106 may receive the signal information associated with one or more events at the plurality of sensor elements 114. The event sensor 124 may include a plurality of pixels, wherein each of the plurality of pixels are sensitive to the light intensity (pixel intensity). The event sensor 124 determines an event based on detecting a change in the light intensity of the reflected light signal. Further, the event sensor 124 generates the signal information based on the detected change in the light intensity of the reflected light signal to be greater than a threshold intensity.

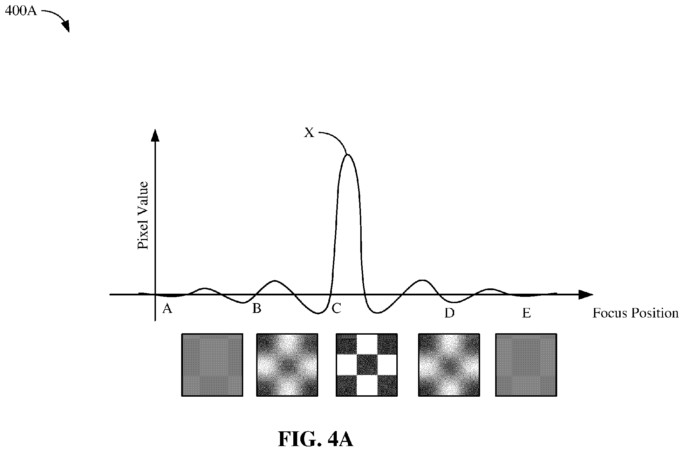

Further, each of the plurality of sensor elements 114 is associated with one or more event sensors. For example, each of the plurality of sensor elements 114 may be pixels of the one or more event sensors. Additionally, each of the plurality of sensor elements 114 may operate asynchronously to capture the reflected light signal to identify the event from the sequence of events. For example, in asynchronous mode of operation, each of the plurality of sensor elements 114 may detect and respond to the identified event independently from a clock signal. Specifically, each of the plurality of sensor elements 114 may operate immediately when the event may be identified. This may facilitate each of the plurality of sensor elements 114 to work independent from each other, in contrast to synchronous operation of the sensor elements, where each of the plurality of sensor elements may be coordinated by the clock signal and perform operations at a fixed interval. This may facilitate immediate processing and transmission upon the identification of the event, thereby minimizing delays. Further, each of the plurality of sensor elements 114 operating asynchronously may remain in low power state until event occurs, thereby reducing power consumption. Details associated with the identification of the event are provided, for example, in FIGs. 4A-4D.

In an example, the at least one light sensor 110 may further include an image sensor 126 configured to capture image frames. In an example, the processor 106 may determine colour information associated with at least the part of the dental object based on at least a first image captured by the image sensor 126. In another example, the image sensor 126 is configured to capture the colour information from at least the part the dental object caused by the visible wavelength, NIR, UV and/or IR wavelength.

The image sensor 126 may include multiple cameras, such as, high speed cameras. In one example, the multiple cameras may be arranged around the projector unit 116 or next to the projector unit 116. In an example, the image sensor 126 may include, but are not limited to a red, green, blue (RGB) sensor, an Infrared (IR) sensor, a Near IR (NIR) sensor, or an ultraviolet (UV) sensor.

The image sensor 126 may include a plurality of pixels, wherein each of a plurality of single-color channels and each of a plurality of combined-colour channels may be aligned to each of the plurality of pixels. In this example, each of the colour channels may overlap with a pixel of the image sensor 126 or with a group of pixels of the image sensor 126.

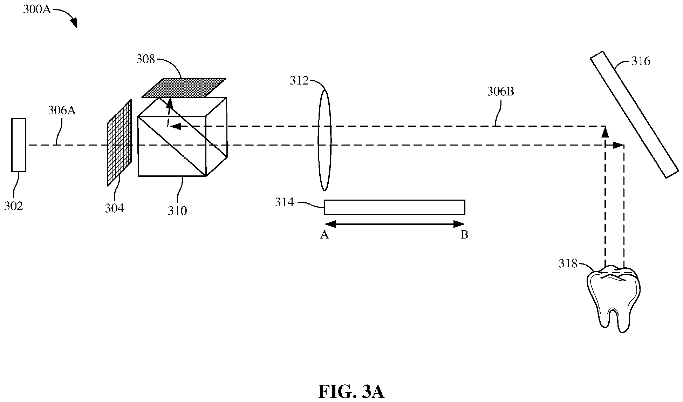

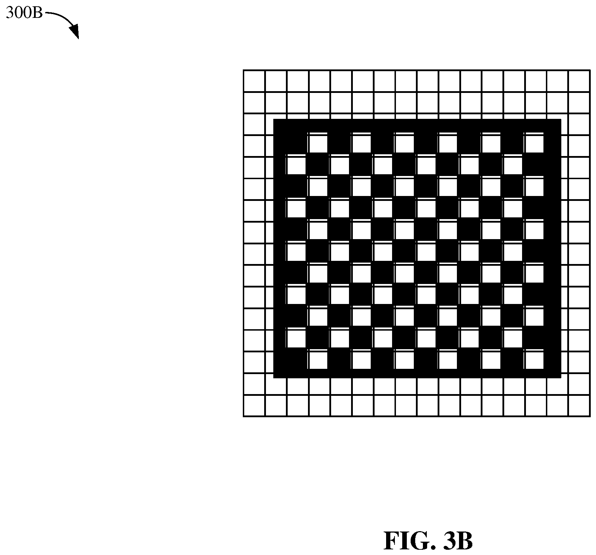

In operation, the projector unit 118 generates the light signal using light sources, for example, LEDs, and lasers. Thereafter, the pattern generator 118 generate the illumination pattern, for example, a checkerboard pattern, that may be projected onto the dental object within the oral cavity. The generated light signal may illuminate at least the part of the dental object, and the reflected light from at least the part of the dental object may be captured by the at least one light sensor 110 though mirrors and lenses. Details associated with the illumination of the dental object are provided in conjunction with, for example, FIG. 3.

Thereafter, the processors 106 are configured to receive the signal information from the plurality of sensor elements 114. In an example, the signal information is associated with one or more events, or one or more sequences of events at the plurality of sensor elements 114. In an example, the signal information includes, but is not limited to, location information and timestamp information. The location information may include information associated with a location of at least the part of the dental object at which the illuminating pattern may focus with respect to a location of the plurality of sensor elements of the event sensor 124 or the light sensor 110. The timestamp information may include information associated with a time period or a time instant at which the illuminating pattern may focus on at least the part of the dental object and/or a time period or a time instant at which an event, i.e., a change in the light intensity greater than the threshold intensity, is detected.

The processors 106 are configured to determine a sequence of events associated with one or more sensor elements from the plurality of sensor elements 114 based on the received signal information. In an example, an event may correspond to detecting a change in the light intensity of the reflected light signal such as, but not limited to an UP event, of a DOWN event. Further, the sequence of events may correspond to detection of one or more events in succession, such as, but not limited to a sequence of UP events or a sequence of DOWN events.

The processors 106 are configured to determine position information of the focus element 112 with respect to at least the part of the dental object based on the sequence of events. The position information may include, but is not limited to, information associated with the focus position of the focus element 112 where the sequence of events may be detected. The processors 106 are configured to generate at least one 3D point associated with at least the part of the dental object for the 3D representation of the dental object based on the position information. Details of the generation of the 3D representation of the dental object are provided for example, in FIGs. 1C, 2, 3A, and 4A-4D.

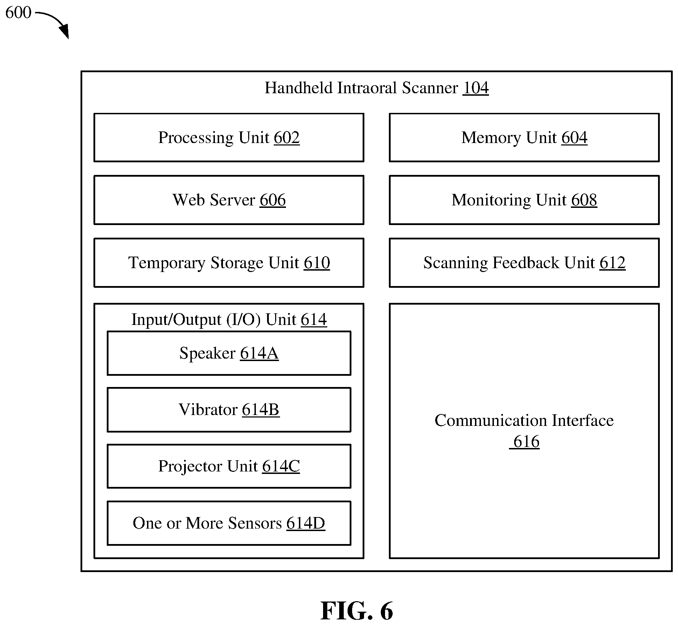

In an example, the hand-held intraoral scanner 104 may include a web server that may be configured to communicate via a web network and establish a connection to the communication channel 108. The hand-held intraoral scanner 104 may be configured to execute the web server to provide the signal information obtained from the plurality of sensor elements to the processors 106. The hand-held intraoral scanner 104 may further include a processing unit, a memory unit, a communication interface, the sensors, and additional components. The processing unit, the memory unit, the communication interface, and the additional components may be communicatively coupled to each other. Details of the components of the hand-held intraoral scanner 104 are further provided, for example, in FIG. 1C and FIG. 6.

FIG. 1B shows an example schematic diagram 124 of the intraoral scanning system 102, in accordance with an example embodiment. The intraoral scanning system 102 includes the hand-held intraoral scanner 104 configured to scan a dental object 128 of a subject. In an example, the hand-held intraoral scanner 104 may emit light of various wavelengths. For example, the images of the dental object 128 may be captured using light having different wavelengths, such as white light, blue light, IR light, NIR light, fluorescent light, etc. To this end, visible light images and/or the IR images for the dental object may be captured from a same position and at same time due to high pulse repetition rate of the light emitted from the hand-held intraoral scanner 104.

The signal information and the colour information generated by the hand-held intraoral scanner 104 may be communicated to the processors 106 over wired or wireless communication channel 108. In an example, the processors 106 may be implemented as a computing device 130A or a server 130B external to the hand-held intraoral scanner 104.

The system 102 includes the one or more processors 106 arranged in the hand-held intraoral scanner 104, the external computer 130A and/or the server 130B. The hand-held intraoral scanner 104 may include a processor, and the processor is configured to process sensor data from sensors of the hand-held intraoral scanner 104 into information, such as the signal information and colour information configured to be transmitted to the external computer 130A or the server 130B. Furthermore, the external computer 130A or the server 130B may include the processors 106 to process the received signal information and colour information.

For example, the subject may require a dental treatment. In such a case, the system 102 may be utilized by a user, such as a dentist to provide the dental treatment to the subject. In an embodiment, the subject may be present at a dental clinic. In such a case, the system 102 may be utilized in a treatment room of the dental clinic. In another embodiment, the subject may have been requested for a home visit for the dental treatment. In such a case, the system 102 may be utilized in the home of the subject. To start the dental treatment, the hand-held intraoral scanner 104 may be utilized by the user to capture the signal information and the colour information of the dental object 128 of the subject, using the at least one light sensor 110.

In an example, throughout a scanning of the dental object of the subject, the projector unit 116 may be configured to constantly emit the light signal 110A (for example, the infrared light signal or the visible light signal).

FIG. 1C illustrates another example schematic diagram of the intraoral scanning system 102, in accordance with an embodiment. According to the present example, the processors 106 of the system 102 receive the signal information 132 and the colour information 134, for example, from the hand-held intraoral scanner 104.

The processors 106 are configured to receive the signal information 132 associated with at least the part of the dental object 128 from the plurality of sensor elements 114. In an example, the processors 106 are configured to obtain the signal information 132 with respect to at least the part of the dental object, when the event may be identified by the plurality of sensor elements 114 based on the reflected light signal 110A from at least the part of the dental object 128.

Further, the processors 106 are configured to determine a sequence of events associated with one or more sensor elements from the plurality of sensor elements 114 based on the received signal information 132. The sequence of events may include one or more events detected based on the change in the light intensity on at least the part of the dental object 128. In an example, the event sensor 124 may detect the light signal 110A and in response to detecting a change in the intensity of the light signal 110A on at least the part of the dental object 128, the event sensor 124 may trigger the event. The event sensor 124 may capture the signal asynchronously, thereby providing high temporal resolution. Further, the event sensor 124 may provide asynchronous binary detection of events, thereby implying no shutter speed needed as each pixel is smart and generates an event only upon detection of the active light signal. Upon the triggering of event, the signal information 132 including the location information and the timestamp information may be generated. The location information may include, but is not limited to, information associated with x, and y coordinates of at least the part of the dental object at which the event is detected.

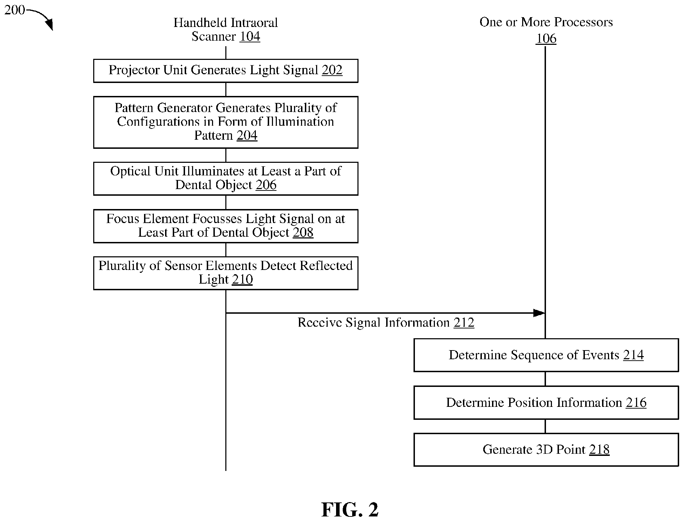

Thereafter, the processors 106 are configured to determine the position information of the focus element 112 with respect to one or more parts of the dental object 128 based on the sequence of events. The position information may include, but is not limited to, information associated with the focus position of the focus element 112. This may facilitate to determine depth information (z coordinate) for at least the part of the dental object 128 for which the event is detected. Details associated with the focus position are provided for example, in FIGs. 2, 3A, and 4A-4D.

Further, the processors 106 are configured to generate at least one 3D point 136 associated with at least the part of the dental object 128 for the 3D representation of the dental object 128 based on the position information. In an example, the at least one 3D point 136 associated with at least the part of the dental object may represent a specific location in a 3D space. The at least one 3D point 136 may have coordinates (x, y, z) that may define a position of the 3D point to a reference frame in a 3D space. It may be noted, the reference frame in the 3D space may be used to define position and orientation of the 3D point in the 3D space. In an example, the 3D point 136 may include information associated with the light intensity, and the timestamp, in addition to the location information (for example, the coordinates) for at least the part of the dental object 128.

In an embodiment, the processors 106 may be configured to determine one or more sequences of events associated with the plurality of sensor elements 114 based on the received signal information. The one or more sequence of events may correspond to multiple sequence of events associated with the one or more sensor elements from the plurality of sensor elements 114 based on the signal information that may be identified with respect to the one or more events. Thereafter, the processors 106 may be configured to determine position information of the focus element 112 with respect to one or more parts of the dental object 128 based on each of the one or more sequences of events. Based on the determined position information, the processors 106 may be configured to generate the 3D point cloud associated with the dental object for the 3D representation of the dental object. In an example, the processors 106 may be configured to generate 3D point for each of the one or more part of the dental object 128. Thereafter, a collection of the 3D points 136 together may represent a 3D point cloud of a shape or surface of the dental object 128. In an example, 3D points generated from one or more sequences of events may be compiled to create the 3D point cloud.

Upon identification of the event, the image sensor 126 may be triggered to capture the first image 138. The processors 106 are configured to determine the colour information 134 associated with at least the part of the dental object 128 based on at least the first image 138 captured by the image sensor 126. In an example, the processors 106 are configured to capture the first image 138 with respect to at least the part of the dental object corresponding to the event. Further, the processors 106 are configured to generate a 3D model 140 of at least the part of the dental object 128 based on the at least one 3D point 136 and the colour information 134 corresponding to the captured first image 138. In an example, the processors 106 are configured to generate the 3D model 140 based on overlaying of the 3D point 136 associated with at least the part of the dental object on the colour information 134 for the corresponding part of the dental object 128.

It may be noted, the 3D model 140 may include 3D surface information that may be represented as a mesh. Further, to overlay the colour information 134 associated with the captured first image 138 onto the at least one 3D point 136, the processors 106 may have to select positions in the 3D model 140 to position or overlay the colour information 134 on the 3D point 136. The selection of the positions for overlaying the colour information 134 ensures that images are positioned at approximately a constant distance from the surface of the dental object 128, as well as positions and directions of placement or overlaying of the colour information 134 are varied smoothly across the 3D model 140.

The event sensor 124 may capture 3D representation of the dental object 128 and generate the 3D point cloud, however they may not facilitate to provide the colour information thereof. In an example, a separate image sensor 126 may be incorporated in the scanner 104 to provide colour/ texture information. In one embodiment the image sensor 126 is a camera residing in the aperture of the scanner 104, as such sharing the same optical path with the event sensor 124. In another embodiment the image sensor 126 is situated outside of the optical path that is used for 3D scanning.

In another example, a hybrid event sensor may be employed by the scanner 104. Such a hybrid event sensor may include an array of RGB pixels in addition to event pixels. For example, a hybrid event sensor may include a 4x4 pixel groups with every 1/16 pixels being a dedicated active event sensor pixel. This gives the possibility to add colour information to the 3D scan using only a single sensor. In such an example, the hybrid event sensor may employ fixed clock for capturing RGB images while using standard event sensing for the event part.

In the example illustrated in FIG. 1C, the system 102 includes a display unit 142. For example, a 3D representation 144 of at least the part of the dental object 128 may be rendered on the display unit 142. The processors 106 are configured to display the 3D representation 144 in real-time. The displayed 3D representation 144 includes both the 3D point and the colour image.

FIG. 2 illustrates a sequence diagram 200 for generating a 3D representation of a dental object, in accordance with an example embodiment. FIG. 2 is explained in conjunction with elements of FIG. 1A, FIG. 1B, and FIG. 1C. The sequence diagram 200 may include the hand-held intraoral scanner 104 and the one or more processors 106. The sequence diagram 200 may depict operations performed by at least one of the hand-held intraoral scanner104 and the processors 106.

At 202, the projector unit 116 of the hand-held intraoral scanner 104 may generate the light signal. The light signal emitted by the projector unit 116 may facilitate the pattern generator 118 to generate the plurality of configurations.

At 204, the pattern generator 118 may generate, using the generated light signal, the plurality of configurations in form of an illumination pattern. The pattern generator 118 may employ the generated light signal to project structured light signal (e.g., the illumination pattern) onto at least the part of the dental object 128, thereby illuminating thereon. The plurality of configurations may correspond to one or more patterns or arrangement of light signals that can be projected onto the dental object 128 in form of the illumination pattern. The illuminating pattern may include an array of segments to achieve a spot illumination. Such an illumination pattern may enhance accuracy and resolution of the captured signal information

At 206, the optical unit 120 may transmit the light signal towards the dental object 128 along an optical path by illuminating at least the part of the dental object with the generated light signal. Thereafter, the optical unit 120 may transmit the reflected light signal from the dental object 128 to the at least one light sensor 110.

At 208, the focus element 112 may focus the light signal on at least the part of the dental object 128. The focus element 112 may focus or direct the light signal onto at least the part of the dental object 128 which needs to be illuminated. In an example, the focus element 112 may gathers and concentrates the light signal from the projector unit 116 and directs the concentrated light onto at least the part of the dental object 128 within the oral cavity, thereby ensuring that the illuminating pattern is sharp, well-defined, and properly focussed on at least the part of the dental object 128. This may allow the scanner 104 to achieve high resolution and better-quality imaging.

At 210, the plurality of sensor elements 114 may detect the reflected light signal from at least the part of the dental object 128. In an example, the plurality of sensor elements 114 may capture the reflected light signal and convert them into the signal information 132 associated with one or more events at the plurality of sensor elements 114.

At 212, the signal information 132 from the plurality of sensor elements 114 may be received by the processors 106. As mentioned above, the signal information 132 may include the location information, and the timestamp information corresponding to at least the part of the dental object 128 where the event is detected.

At 214, the sequence of events associated with one or more sensor elements from the plurality of sensor elements 114 may be determined based on the received signal information. Details associated with the determining the sequence of events are provided, for example, in FIGs 3, and 4A-4D.

At 216, the position information of the focus element 112 with respect to at least the part of the dental object 128 may be determined based on the sequence of events. The position information of the focus element 112 may include a distance of the focus element 112 from the scanner 104. The processors 106 may be configured to control a movement of the focus element 112 to project the illumination pattern onto the dental object 128. Further, a position of the focus element 112 may be determined when the illuminating pattern is sharp, well-defined, and properly focussed on at least the part of the dental object 128. This may facilitate the scanner 104 to determine the position information of the focal element 112 from the scanner 104. Specifically, the position information of the focal element 112 may include a distance between the scanner 104 and the dental object 128. Further, the movement of the focus element 112 changes a depth associated with at least one of the plurality of sensor elements 114 for capturing the reflected light signal. Based on the position information and the depth of focus, the processors 106 may generate the at least one 3D point 136 associated with at least the part of the dental object 128.

The position information of the focus element 112 may be determined by spatial correlation corresponding to a statistical measure that quantifies a degree of similarity between two regions within an image. This may facilitate to assess sharpness by determining correlation between the pixel values in overlapping regions of the image. For example, a sequence of images of at least the part of the dental object 128 may be captured at different positions of the focus element 112. The processors 106 may select one or more regions for each of the sequence of images to compare for spatial correlation. For example, small overlapping windows may be selected for each consecutive image of the sequence of images. Thereafter, the processors 106 may determine a similarity between the pixel values in the selected regions of the image to calculate spatial correlation between the corresponding regions of the images. This may facilitate to determine resemblance of the corresponding regions of the images with respect to the movement of the focus element 112. In an example, a high correlation value indicates a sharp image, thereby indicating better focus. The processors 106 may determine the position of the focus element 112 in response to the high correlation value. For example, a depth information per pixel or a group of pixels of the light sensor 110 may be assigned corresponding to the maximum signal (or the high correlation value) obtained on the pixel or the group of pixels following a movement of the illumination pattern onto the dental object 128 with respect to the movement of the focus element 112.

At 218, at least one 3D point associated with at least the part of the dental object for the 3D representation of the dental object may be generated based on the position information. Details associated with the determining the sequence of events are provided, for example, in FIG 4A-4D.

FIG. 3A illustrates a schematic diagram 300A of an optical path followed by a light signal, in accordance with various example embodiments. FIG. 3A is explained in conjunction with elements of FIG. 1A, FIG. 1B, FIG. 1C, and FIG. 2. The schematic diagram 300A may include an optical path 306A followed by the generated light signal and an optical path 306B followed by the reflected light signal.

In an example, a projector unit 302 may generate the light signal. The light signal passes through a beam splitter 310 via an illuminating pattern 304. The illuminating pattern 304 is a checkerboard pattern, as explained for example, in FIG. 3B. In an example, the illuminating pattern 304 may be disposed on a transparent base for example, a glass plate. The projector unit 302 may be for example, a LED light source with a reflector behind it to direct the generates light signal through the illuminating pattern 304.

Thereafter, the light signal is passed through a focus element 312 to focus the light signal into a beam that may focus onto at least the part of the dental object 318. The processor 106 may be configured to control a movement of the focus element 312 to project the illumination pattern. As shown in the FIG. 3A, the focus element 312 may move back and forth from a position A to B in a horizontal direction along a mechanical actuator 314. This may facilitate moving the focus of the image in 3D space. In an example, the focus element 312 may correspond to a movable focal lens that can be moved along the optical path to change focal length of the optical unit. With the movement of the focus element 312, the illumination pattern 304 that may be projected onto the dental object may also move. Further, a position of the focus element 312 may be determined when the illuminating pattern is sharp, well-defined, and properly focussed on at least the part of the dental object. This may facilitate the scanner 104 to determine the position information of the focal element from the scanner 104. Specifically, the position information of the focal element may include a distance between the scanner 104 and the dental object. Further, the movement of the focus element 312 changes a depth associated with at least one of the plurality of sensor elements for capturing the reflected light signal. Based on the position information and the depth of focus, the processor 106 may generate a 3D point associated with at least the part of the dental object. The examples of the focus element 312 may include, but are not limited to, a zoom lens.

The beam is reflected by a mirror 316 and directed towards a field of view such as at least the part of the dental object 318. Thereafter, the light signal is reflected back from the dental object to the at least one light sensor 308 via the optical path 306B.

The beam splitter 310 may be configured to transmit the light signal from the projector unit 302 to the dental object 318 and reflect the light signal returned from the dental object 318 to the at least one light sensor 308. For example, the beam splitter 310 may be a polarization beam splitter (PBS).

FIG. 3B illustrates an exemplary illuminating pattern 300B, in accordance with various example embodiments. FIG. 3B is explained in conjunction with elements of FIG. 1A, FIG. 1B, FIG. 1C, FIG. 2, and FIG. 3A. The illuminating pattern 300B may correspond to a checkerboard pattern. It is to be noted that a variety of patterns can be used in addition to the checkerboard pattern without any deviation in the scope of the disclosure.

The illuminating pattern 300B may be include an array of segments to achieve spot-illumination equivalent. The illumination spots through the pattern can be nearly diffraction limited. For example, the illuminating pattern 300B can include an array of segments that have a diameter of about 1 μm, 10 μm, 25 μm, 50 μm, 1 mm or 2 mm or any values therebetween. For example, the size of the pinholes may be configured adapted to the numerical aperture (NA) of the optical unit and the wavelength of the light signal. For example, the size of the detection pinholes may be further adapted to a magnification of the optical unit 120.

With reference to FIG. 3B, there is shown a checkerboard pattern including alternating light and dark squares, similar to a chessboard. This facilitates to provide high-contrast and well-defined edges. Specifically, the checkerboard pattern includes an array of white (light) squares, and black (dark) squares arranged in a grid pattern. For example, the checkerboard pattern may include, but not limited to 8x8 grid, or 10x10 grid. Each square in the checkerboard pattern is of equal size, thereby ensuring uniformity. For example, in a 10x10 grid, each square may correspond to 20x20 pixels. In an example, the pixel value may range from 0 (black) to 255 (white) in an 8-bit grayscale image. In another example, the pixel value may correspond to -1 for black squares and +1 for white squares. Further, the processors 106 may generate the dark (black) square based on the detected change in the light intensity of the reflected light signal to be less than the threshold intensity. On the contrary, the processors 106 may generate the light (white) square based on the detected change in the light intensity of the reflected light signal to be greater than the threshold intensity.

FIG. 4A illustrates an example graphical representation 400A indicating a change in light intensity with respect to a focus position associated with the focus element 112, in accordance with various example embodiments. FIG. 4A is explained in conjunction with elements of FIG. 1A, FIG. 1B, FIG. 1C, FIG. 2, FIG. 3A, and FIG. 3B. The graphical representation 400A may depict a graph between the focus position of the focus element 112 on x-axis and corresponding pixel values on y-axis.

In an example, the sequence of images may be captured by the at least one light sensor 110 at different focus positions, for example, but not limited to, a first position A, a second position B, a third position C, a fourth position D, and a fifth position E. For each position a pixel value may be determined. For example, a pixel value may correspond to a sharpness measure of each of the sequence of images. The pixel value may be determined based on the following formula:

$$A = \sum_{i=1}^{NxN} f(i) \, I$$

where, $A$ denotes amplitude of the pixel value, $N \times N$

denotes a grid size, for example, not limited to 6x6, *f* denotes weight function for a given square of the illumination pattern, and *I* denotes a light intensity value for a corresponding square of the illumination pattern.

With reference to FIG. 4A, a pixel value for a first square of the 6x6 grid at different positions may be determined. As shown, the illumination pattern is out of focus for the first position A. Further, the focus element 112 may be moved to the second position B, the pixel value for the first square of the 6x6 grid at the second position B is high as compared to the pixel value at first position A, thereby providing a blur illumination pattern. Thereafter, the focus element 112 may be moved to the third position C, the pixel value for the first square of the 6x6 grid at the third position C is higher as compared to the pixel value at the second position B, thereby providing a sharp illumination pattern. Further, the focus element 112 may be moved to the fourth position D, the pixel value for the first square of the 6x6 grid at the fourth position D is low as compared to the pixel value at third position C, thereby providing a blur illumination pattern again. Further, the focus element 112 may be moved to the fifth position E, the pixel value for the first square of the 6x6 grid at the fifth position E is lower as compared to the pixel value at fourth position D, thereby providing an out of focus illumination pattern. The graphical representation 400A displays a peak X at the focus position where the image is sharpest, and an inverse peak for a no event

In an embodiment, the processors 106 may be configured to determine a peak value of light intensity associated with the reflected light signal and generate the at least one 3D point 136 associated with at least the part of the dental object 128 based on the peak value. In an example, the peak value may correspond to highest value of the light intensity associated with the reflected light signal when the event may be identified. This may facilitate to generate a focused image of at least the part of the dental object 128. Thereafter, the processors 106 may be configured to determine the position information of the focus element 112 with respect to at least the part of the dental object 128 and generate the at least one 3D point 136 associated with at least the part of the dental object 128. Traditionally, the spatial correlation measures are used to determine focus quality of an image or a region within an image. This enables to measure and quantify similarity or relationship between neighbouring pixels or regions in the image. In operation, when the focus element moves back-and-forth to focus the illuminating pattern onto the dental object 128, the pixel values of each pixel of the image may change. Further, when the illumination pattern is sharp or in-focus, the event may be determined.

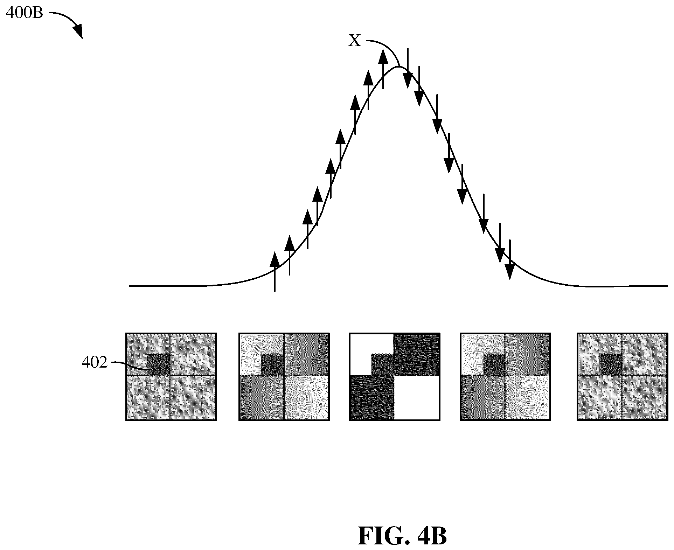

FIG. 4B illustrates another example graphical representation 400B of the peak X of graphical representation 400A with respect to the detected event, in accordance with various example embodiments. FIG. 4B is explained in conjunction with elements of FIG. 1A, FIG. 1B, FIG. 1C, FIG. 2, FIG. 3A, FIG. 3B, and FIG. 4A. The graphical representation 400B may depict the peak X of graphical representation 400A at the third position C of the focus element 112. The graphical representation 400B further includes the illumination pattern 304 along with the event 402 to be detected.

In an embodiment, the processors 106 may be configured to compare a first value of light intensity of the reflected light signal for at least one sensor element from the plurality of sensor elements 114 with a first threshold value for the corresponding at least one sensor element. The threshold value may correspond to a numeric value between the pixel value. In an example, for a Grayscale image the pixel value may range from 0 (black) to 255 (white), the first threshold value may correspond to a numeric value between the range, for example, 0-255. When the first value of light intensity of the reflected light signal is greater or lesser than the first threshold value, the processors 106 may be configured to identify an event from the sequence of events associated with the at least one sensor element based on the comparison. For example, if the first value of light intensity of the reflected light signal is greater than the first threshold value, an UP event may be identified, depicted by an UP arrow. On the contrary, if the first value of light intensity of the reflected light signal is lesser than the first threshold value, a Down event may be identified, depicted by a Down arrow.

As shown, a first square depicted as a dark or grey or out of focus illumination pattern denote a lack of event. For example, the first value of light intensity of the reflected light signal may be equal to the first threshold value for the corresponding focus position. Further, a subsequent square depicted as a light or less grey or blur illumination pattern denote a UP event. For example, the first value of light intensity of the reflected light signal may be greater than the first threshold value for the corresponding focus position. Followed by a series of UP events, a subsequent square depicted as a white or sharp illumination pattern denote a peak event. For example, the first value of light intensity of the reflected light signal may be highest for the corresponding focus position, thereby generating the at least one 3D point associated with at least the part of the dental object based on the peak value.

Thereafter, a subsequent square depicted as a light or less grey or blur illumination pattern denote a Down event. For example, the first value of light intensity of the reflected light signal may be less than the first threshold value for the corresponding focus position. Followed by a series of Down events, a subsequent square depicted as a dark or grey or out of focus illumination pattern denote the lack of event.

In an example, the light sensor 110 may correspond to the hybrid event sensor with 4x4 pixel groups where every 1/16 pixels being a dedicated active event sensor pixel. In such an example, the light sensor 110 may reset the RGB shutters after receiving a sequence of UP events in a row and close it after receiving a sequence of DOWN events in a row. This would effectively cause the sensor to take an image exactly at the peak of the amplitude curve.

In an embodiment, the processors 106 may be further configured to determine a second threshold value and a third threshold value for the at least one sensor element based on the first value of light intensity in response to identifying an event from the sequence of events associated with the at least one sensor element. The second threshold value may correspond to a numeric value less than the first value of light intensity, and the third threshold value may correspond to a numeric value greater than the first value of light intensity. Thereafter, the processors 106 may be configured to determine a second value of light intensity associated with a second reflected light signal captured after the reflected light signal. The second value of light intensity may correspond to a current light intensity level associated with a subsequent pixel where the plurality of sensor elements 114 detects the second reflected light signal with respect to a second event in the sequence of events.

The second value may be compared with each of the second threshold value and the third threshold value for the at least one sensor element and identify another event from the sequence of events associated with the at least one sensor element based on the comparison. For example, if the second value of light intensity of the reflected light signal is greater than the second threshold value or the third threshold value, an UP event may be identified, depicted by an UP arrow. On the contrary, if the second value of light intensity of the reflected light signal is lesser than the second threshold value or the third threshold value, a Down event may be identified, depicted by a Down arrow. In an example, if the second value go past one of the second threshold value and the third threshold value, another event may be identified. In such an example, another event may correspond to the UP or the DOWN event depending on a direction of the change of the light intensity value. For example, if the second value is less than the second threshold value (that may be less than the first value and the third threshold value), a DOWN event may be identified. In an example, if the second value is greater than the second threshold value (that may be less than the first value and the third threshold value), a DOWN event may be identified. Further, if the second value is less than the third threshold value (that may be greater than the first value and the second threshold value), a UP event may be identified. In an example, if the second value is greater than the third threshold value (that may be greater than the first value and the second threshold value), an UP event may be identified.

In an embodiment, the processors 106 may be configured to determine a frequency of the sequence of events. The frequency of the sequence of events may correspond to rate of events for example, up event or down event. Further, the processors 106 may be configured to compare the frequency of the sequence of events with a threshold frequency and in response to determining the frequency to be greater than the threshold frequency, determine the position information of the focus element 112 with respect to at least the part of the dental object 128. For example, if the frequency is greater than the threshold value, the position information of the focus element 112 with respect to at least the part of the dental object 128 may be determined. On the contrary, if the frequency is less than the threshold value, the position information of the focus element 112 with respect to at least the part of the dental object 128 may not be determined. In an example, the frequency of the sequence of events may allow to determine at least the part of the dental object 128 where highest density of relevant events may be located, thereby guiding the focus element 112 to that part of the dental object 128. This may facilitate in real-time adjustments to the position of the focus element 112 to achieve focus.

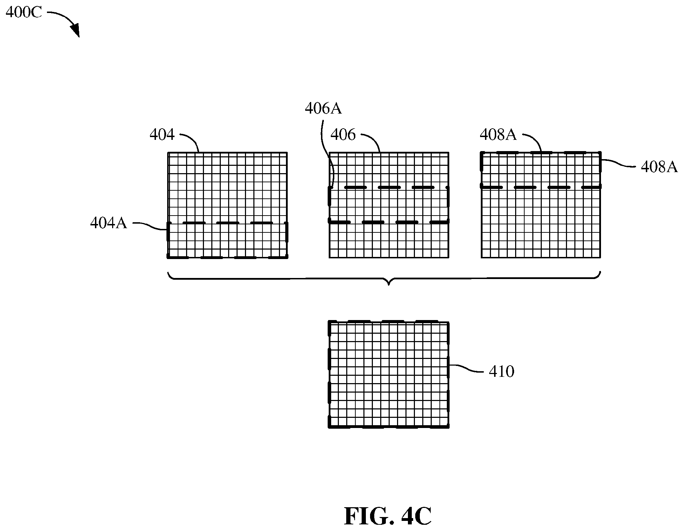

FIG. 4C illustrates an exemplary diagram 400C of image frames captured by the image sensor 126, in accordance with various example embodiments. FIG. 4C is explained in conjunction with elements of FIG. 1A, FIG. 1B, FIG. 1C, FIG. 2, FIG. 3A, FIG. 3B, FIG. 4A and FIG. 4B. The diagram 400C may depict the image frames, for example, 404, 406, and 408, captured by the image sensor 126 for different focus positions.

In an example, the processors 106 may be configured to determine, using the event sensor 124, a first event (for example, the event 402) of the sequence of events at one or more of the plurality of senor elements 114. Thereafter, the image sensor 126 may be triggered to capture a first image with respect to at least the part of the dental object 128 corresponding to the first event. Further, the processors 106 may be configured to determine colour information associated with at least the part of the dental object 128 based on at least the first image captured by the image sensor 126.

Traditionally, the scanner may be moved over the oral cavity, thereby capturing a series of images at different depths. This may require significant memory allocation and computational power. However, the proposed scanner 104 may trigger the light sensor 110 to capture the image frames 404, 406, and 408 with respect to at least the part of the dental object corresponding to the first event. For each of the image frames 404, 406, and 408, calculate sub-image frames 404A, 406A, and 408A where the event is detected. Further, the FPGA may perform correlation calculations on the sub-image frames 404A, 406A, and 408A of the captured image frames 404, 406, and 408 to determine focal planes for each pixel. Further, each of the sub-image frames 404A, 406A, and 408A may be aligned to create a sub-image 410 that may be in focus onto the at least the part of the dental object where the event is detected. This may create the 3D model of at least the part of the dental object based on the 3D point generated based on, for example, the first event, and the colour information captured in, for example, the first image. In such a case the intraoral scanner 104 may calculate focus for each pixel in only the sub-image frames 404A, 406A, and 408A, thereby enabling saving time and resources as compared to the traditional scanning method, thereby mitigating significant bottleneck in the scanning process. Further, the amount of data gathered may significantly reduce in the scanning process, thereby reducing processing power and memory allocation requirements. In an example, the FPGA may be a heat-generating and expensive component, therefore removing such a component from the scanner 104 may result in creating a compact, less power consuming, and less expensive scanning device.

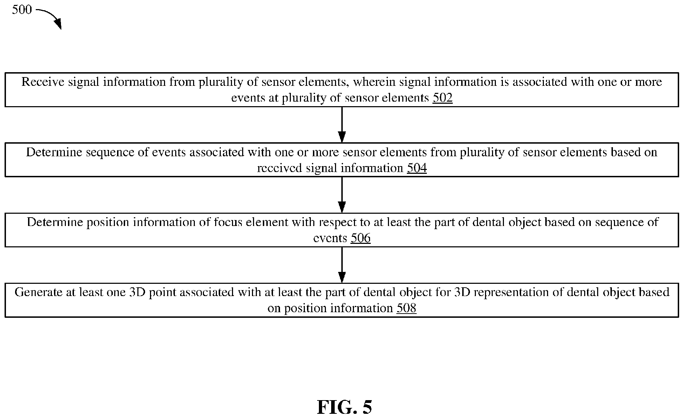

FIG. 5 illustrates a flowchart 500 of a method for generating the 3D representation of the dental object, in accordance with an example embodiment. FIG. 5 is explained in conjunction with elements of FIG. 1A, FIG. 1B, FIG. 1C, FIG. 2, FIG. 3, FIG. 4A, FIG. 4B, and FIG. 4C. The operations of the exemplary method may be executed by any computing system, for example, by the processors 106 of the system 102 of FIG. 1. The operations of the flowchart 500 may start at 502

At 502, the signal information 132 from the plurality of sensor elements 114 may be received. The signal information 132 may be associated with one or more events at the plurality of sensor elements 114.

At 504, a sequence of events associated with one or more sensor elements from the plurality of sensor elements 114 may be determined based on the received signal information 132. In an embodiment, the processors 106 may be configured to compare a first value of light intensity of the reflected light signal for at least one sensor element from the plurality of sensor elements 114 with a first threshold value for the corresponding at least one sensor element and identify an event from the sequence of events associated with the at least one sensor element based on the comparison.

At 506 position information of the focus element 112 with respect to at least the part of the dental object 128 may be determined based on the sequence of events. In an embodiment, the processors 106 may be configured to determine a frequency of the sequence of events and compare the frequency of the sequence of events with a threshold frequency. Thereafter, the processors 106 may be configured to determine the position information of the focus element with respect to at least the part of the dental object, in response to determining the frequency to be greater than the threshold frequency.

At 508 at least one 3D point 136 associated with at least the part of the dental object 128 for the 3D representation of the dental object 128 may be generated based on the position information.

Accordingly, blocks of the flowchart 500 support combinations of means for performing the specified functions and combinations of operations for performing the specified functions. It will also be understood that one or more blocks of the flowchart 500 can be implemented by special purpose hardware-based computer systems which perform the specified functions, or combinations of special purpose hardware and computer instructions.

Alternatively, the intraoral scanner 104 may include means for performing each of the operations described above. In this regard, according to an example embodiment, examples of means for performing operations may include, for example, a processor and/or a device or circuit for executing instructions, such as the operations or instructions for controlling heat transfer in the hand-held intraoral scanner 104.

FIG. 6 illustrates a block diagram 600 of the hand-held intraoral scanner 104, in accordance with an example embodiment. FIG. 6 is explained in conjunction with elements of IG. 1A, FIG. 1B, FIG. 1C, FIG. 2, FIG. 3, FIG. 4A, FIG. 4B, FIG. 4C, and FIG. 5. The hand-held intraoral scanner 104 may include at least one processing unit (hereinafter, also referred to as "processing unit 602"), a memory unit 604, a web server 606, a monitoring unit 608, a temporary storage unit 610, a scanning feedback unit 612, an input/output (I/O) unit 614, and a communication interface 616.

The processing unit 602 may be embodied in a number of different ways. For example, the processing unit 602 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. In an embodiment, the processing unit 602 may be embodied as a high-performance microprocessor having series of System on Chip (SOCs) which includes relative powerful and power-efficient Graphics Processing Units (GPUs) and Central Processing Units (CPUs) and a small form factor. As such, in some embodiments, the processing unit 602 may include one or more processing cores configured to perform independently. A multi-core processor may enable multiprocessing within a single physical package. Additionally, or alternatively, the processing unit 602 may include one or more processors configured in tandem via the bus to enable independent execution of instructions, pipelining and/or multithreading.

In some embodiments, the processing unit 602 may be configured to detect light signal (for example, NIR and/or visible light), using the I/O unit 614 during the scanning session of teeth of a subject, such as a patient requiring a dental treatment. The detected light signal may be used to determine the sequence of events The detected NIR and visible light may be used to generate the plurality of images of the dental object 128 or teeth of the subject from various angles or viewing points. In an example embodiment, the processing unit 602 may be in communication with the memory unit 604 via a bus for passing information among components of the scanner 104.

The memory unit 604 may be non-transitory and may include, for example, one or more volatile and/or non-volatile memories. In other words, for example, the memory unit 604 may be an electronic storage device (for example, a computer readable storage medium) comprising gates configured to store data (for example, bits) that may be retrievable by a machine (for example, a computing device like the processing unit 602). The memory unit 604 may be configured to store information, data, content, applications, instructions, or the like, for enabling the apparatus to carry out various functions in accordance with an example embodiment of the present disclosure. For example, the memory unit 604 may be configured to store the detected light signal after the scanning session of the teeth is finished. The detected light signal after the scanning session may be stored as signal information and colour information. In certain cases, the memory unit 604 may be configured to store compressed the signal information and the colour information. As exemplarily illustrated in FIG. 6, the memory unit 604 may be configured to store instructions for execution by the processing unit 602. As such, whether configured by hardware or software methods, or by a combination thereof, the processing unit 602 may represent an entity (for example, physically embodied in circuitry) capable of performing operations according to an embodiment of the present disclosure while configured accordingly. Thus, for example, when the processing unit 602 is embodied as the microprocessor, the processing unit 602 may be specifically configured hardware for conducting the operations described herein. Alternatively, as another example, when the processing unit 602 is embodied as an executor of software instructions, the instructions may specifically configure the processing unit 602 to perform the algorithms and/or operations described herein when the instructions are executed. The processing unit 602 may include, among other things, a clock, an arithmetic logic unit (ALU) and logic gates configured to support operation of the processing unit 602.

The web server 606 may be a software, a hardware, or a combination thereof that may be configured to store and provide data to a web browser associated with the processors 106. For example, the signal information and the colour information be provided to the web browser of the processors 106 via the web server 606. As the web server 606 may be accessed by any web browser, the need for installation of an additional software by the processors 106, to connect to the web server 606 may be eliminated. The web server 606 may communicate to one of the communication channels 108 via a web network. In an example, the web server 606 and the processors 106 may communicate to a common wireless full-duplex communication channel via the web network for transmission and reception of the visible light information and the IR information. The web server 606 and the web browser may communicate via, for example, Hypertext Transfer Protocol (HTTP), Simple Mail Transfer Protocol (SMTP), or File Transfer Protocol (FTP). Once the web server 606 and the web browser are connected, the web server 606 may provide a web application on the web browser.

The monitoring unit 608 may be a software, a hardware, or a combination thereof that may be configured to monitor a bandwidth of one of the communication channels 108 (such as the wireless full-duplex communication channel) via which the scanner 104 and the processors 106 may be connected. Moreover, the monitoring unit 608 may be configured to monitor a connection of one of the communication channels 108 via which the scanner 104 and the processors 106 may be connected.

The temporary storage unit 610 may be a software, a hardware, or a combination thereof that may be configured to store the signal information and colour information when the bandwidth of the communication channels 108 (such as the wireless full-duplex communication channel) is determined to be below the minimum bandwidth. The temporary storage unit 610 may further transmit the stored the signal information and colour information to the processors 106 when the bandwidth is determined to be above or equal the minimum bandwidth. Examples of the temporary storage unit 610 may include, but may not be limited to, a random-access memory (RAM), or a cache memory.

The scanning feedback unit 612 may be a software, a hardware, or a combination thereof that may be configured to receive status input from the monitoring unit 608. Based on the received status input, the scanning feedback unit 612 may provide a scanning feedback signal to the user, such as a dentist, of the hand-held intraoral scanner 104. In an embodiment, the scanning feedback signal is used to provide guidance to the user regarding an area of the teeth where a scanning quality of the scanning session is low and adequate visible light information and/or the NIR information of the is not received. For example, the scanning feedback unit 612 may provide the scanning feedback signal as, for example, an acoustic feedback signal, a haptic feedback, or a visual feedback.

The I/O unit 614 may include circuitry and/or software that may be configured to provide output to the user of the hand-held intraoral scanning device 104 and receive, measure or sense input information. The I/O unit 614 may include a speaker 614A, a vibrator 614B, a projector unit 614C, and one or more sensors 614D. In an embodiment, the speaker 614A may be configured to output the acoustic feedback signal to guide the user. The vibrator 614B may be, for example, a transducer configured to convert the scanning feedback signal that may be an electrical signal into a mechanical output, such as the haptic feedback in form of vibrations to guide the user.

As may be understood, the scanner 104 may be configured to detect the IR and the visible light that may be reflected from the teeth of the subject. In this regard, the projector unit 614C may be configured to output one or more visible or white coloured wavelength pulses, and one or more IR wavelength pulses. For example, the visible wavelength pulses and the IR wavelength pulses may be casted onto the dental object 124 or the teeth to illuminate the teeth of the subject, such as a patient. Further, the visible light wavelength pulses and the IR wavelength pulses may be reflected or refracted from the surface and/or the inner region of the teeth. The one or more sensors 614D may be configured to detect visible coloured wavelength pulses and IR wavelength pulses that may be reflected and/or refracted from the surface or inner region of the teeth. In an example, the one or more sensors 614D may include one or more light sensors, such as event sensor or image sensor. For example, the image sensors may correspond to a camera that may be configured to generate the visible light images 114 and the IR images 112 based on the illumination of the teeth using the IR and the visible light, and the event sensor may correspond to an event camera that may be configured to determine an event,

The communication interface 616 may comprise input interface and output interface for supporting communications to and from the hand-held intraoral scanner 104. The communication interface 616 may be a device or circuitry embodied in either hardware or a combination of hardware and software that is configured to receive and/or transmit data to/from the scanner 104. In this regard, the communication interface 616 may include, for example, an antenna (or multiple antennae) and supporting hardware and/or software for enabling communications with a wireless communication network. Additionally, or alternatively, the communication interface 616 may include the circuitry for interacting with the antenna(s) to cause transmission of signals via the antenna(s) or to handle receipt of signals received via the antenna(s). In some environments, the communication interface 616 may alternatively or additionally support wired communication. As such, for example, the communication interface 616 may include a communication modem and/or other hardware and/or software for supporting communication via cable, digital subscriber line (DSL), universal serial bus (USB) or other mechanisms.

Many modifications and other embodiments of the disclosure set forth herein will come to mind of one skilled in the art to which these disclosures pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

ITEM

1. An intraoral scanning system (102) for generating a three-dimensional (3D) representation of a dental object, the intraoral scanning system comprising:

a hand-held intraoral scanner (104) comprising at least one light sensor (110), wherein the at least one light sensor comprises a focus element (112) to focus a light signal (110A) on at least a part of the dental object (128), and a plurality of sensor elements (114) to detect a reflected light signal from at least the part of the dental object; and

one or more processors (106) connected to the hand-held intraoral scanner, wherein the one or more processors are configured to:

receive signal information (132) from the plurality of sensor elements, wherein the signal information is associated with one or more events at the plurality of sensor elements;

determine a sequence of events associated with one or more sensor elements from the plurality of sensor elements based on the received signal information;

determine position information of the focus element with respect to at least the part of the dental object based on the sequence of events; and

generate at least one 3D point (136) associated with at least the part of the dental object for the 3D representation of the dental object based on the position information.

2. The intraoral scanning system (102) according to item 1, wherein the hand-held intraoral scanner (104) further comprises:

a projector unit (116) to generate the light signal (110A);

a pattern generator (118) to generate, using the generated light signal, a plurality of configurations in form of an illumination pattern (304); and

an optical unit (120) for transmitting the light signal towards the dental object along an optical path by illuminating at least the part of the dental object with the generated light signal, and for transmitting the reflected light signal from the dental object to the at least one light sensor.

3. The intraoral scanning system (102) according to item 2, wherein the illumination pattern (304) is a checkerboard pattern, and wherein the one or more processors (106) are further configured to:

compare a first value of light intensity of the reflected light signal for at least one sensor element from the plurality of sensor elements (114) with a first threshold value for the corresponding at least one sensor element; and

identify an event from the sequence of events associated with the at least one sensor element based on the comparison.

4. The intraoral scanning system (102) according to the item 3, wherein the one or more processors (106) are further configured to identify the event based on at least one of:

in response to determining the first value to be equal to the first threshold value, identify a lack of event for the at least one sensor element,

in response to determining the first value to be greater than the first threshold value, identify an up event for the at least one sensor element, and

in response to determining the first value to be less than the first threshold value, identify a down event for the at least one sensor element.

5. The intraoral scanning system (102) according to item 3 or 4, wherein the one or more processors (106) are further configured to:

in response to identifying an event from the sequence of events associated with the at least one sensor element, determine a second threshold value and a third threshold value for the at least one sensor element based on the first value of light intensity;

determine a second value of light intensity associated with a second reflected light signal captured after the reflected signal

compare the second value with each of the second threshold value and the third threshold value for the at least one sensor element; and

identify another event from the sequence of events associated with the at least one sensor element based on the comparison.

6. The intraoral scanning system (102) according to any of the items 2-5, wherein the one or more processors (106) are further configured to:
control a movement of the focus element (112) to project the illumination pattern (304), wherein the movement of the focus element changes a depth associated with at least one of the plurality of sensor elements (114) for capturing the reflected light signal.

7. The intraoral scanning system (102) according to any of the previous items, wherein the one or more processors (106) are further configured to:

determine a frequency of the sequence of events;

compare the frequency of the sequence of events with a threshold frequency; and

in response to determining the frequency to be greater than the threshold frequency, determine the position information of the focus element (112) with respect to at least the part of the dental object (128).

8. The intraoral scanning system (102) according to any of the previous items, wherein the one or more processors (106) are further configured to:

determine one or more sequences of events associated with the plurality of sensor elements (114) based on the received signal information (132);

determine position information of the focus element (112) with respect to one or more parts of the dental object (128) based on each of the one or more sequences of events; and

generate a 3D point cloud associated with the dental object for the 3D representation of the dental object based on the position information for each of the one or more sequences of events.

9. The intraoral scanning system (102) according to any of the previous items, wherein the at least one light sensor (110) comprises an event sensor (124) configured to generate the signal information (132) based on detecting a change in a light intensity of the reflected light signal to be greater than a threshold intensity, and an image sensor (126) configured to capture image frames.

10. The intraoral scanning system (102) according to the item 9, wherein the image sensor (126) comprises at least one of: a red, green, blue (RGB) sensor, an Infrared (IR) sensor, a Near IR sensor, or an ultraviolet (UV) sensor.

11. The intraoral scanning system (102) according to any of the items 9 or 10, wherein the one or more processors (106) are further configured to determine, using the event sensor (124), a first event of the sequence of events at one or more of the plurality of sensor elements (114) that triggers the image sensor (126) to capture a first image with respect to at least the part of the dental object (128) corresponding to the first event.

12. The intraoral scanning system (102) according to any of the items any 9-11, wherein the one or more processors (106) are further configured to:

generate the at least one 3D point (136) associated with at least the part of the dental object (128) based on the sequence of events detected by the event sensor (124);

determine colour information (134) associated with at least the part of the dental object based on at least the first image captured by the image sensor (126); and

generate a 3D model (140) of at least the part of the dental object based on the 3D point and the colour information.

13. The intraoral scanning system (102) according to any of the previous items, wherein each of the plurality of sensor elements (114) is associated with one or more event sensors, and wherein each of the plurality of sensor elements operate asynchronously to capture the reflected light signal to identify an event from the sequence of events.

14. The intraoral scanning system (102) according to any of the previous items, wherein the signal information (132) comprises at least location information and timestamp information.

15. The intraoral scanning system (102) according to any of the previous items, wherein the one or more processors (106) are further configured to:

determine a peak value of light intensity associated with the reflected light signal; and

generate the at least one 3D point associated with at least the part of the dental object (128) based on the peak value.

16. A method (500) for generating a three-dimensional (3D) representation of a dental object (128), the method being implemented using an intraoral scanning system (102) comprising a hand-held intraoral scanner (104) comprising at least one light sensor (110), wherein the at least one light sensor comprises a focus element (112) to focus a light signal on at least a part of the dental object, and a plurality of sensor elements (114) to detect a reflected light signal from at least the part of the dental object, the method comprising:

receiving (502) signal information (132) from the plurality of sensor elements, wherein the signal information is associated with one or more events at the plurality of sensor elements;

determining (504) a sequence of events associated with one or more sensor elements from the plurality of sensor elements based on the received signal information;

determining (506) position information of the focus element with respect to at least the part of the dental object based on the sequence of events; and

generating (508) at least one 3D point associated with at least the part of the dental object for the 3D representation of the dental object based on the position information.

17. The method according to item 16, further comprising:

comparing a first value of light intensity of the reflected light signal for at least one sensor element from the plurality of sensor elements (114) with a first threshold value for the corresponding at least one sensor element; and

identifying an event from the sequence of events associated with the at least one sensor element based on the comparison.

18. The method according to item 16 or 17, further comprising:

determining a frequency of the sequence of events;

comparing the frequency of the sequence of events with a threshold frequency; and

in response to determining the frequency to be greater than the threshold frequency, determining the position information of the focus element (112) with respect to at least the part of the dental object (128).

19. The method according to item 16 or 17, further comprising:

determining one or more sequences of events associated with the plurality of sensor elements (114) based on the received signal information (132);

determining a position information of the focus element (112) with respect to at least a part of the dental object (128) based on each of the one or more sequences of events; and

generating a 3D point cloud associated with the dental object for the 3D representation of the dental object based on the position information for each of the one or more sequences of events.

20. A computer programmable product comprising a non-transitory computer readable medium having stored thereon computer executable instructions, which when executed by a processing circuitry, cause the processing circuitry to carry out operations, the operations comprising:

receiving signal information (132) from a plurality of sensor elements (114), wherein the signal information is associated with one or more events at the plurality of sensor elements;

determining a sequence of events associated with one or more sensor elements from the plurality of sensor elements based on the received signal information;

determining position information of a focus element (112) with respect to at least a part of a dental object (128) based on the sequence of events; and

generating at least one 3D point associated with at least the part of the dental object for a 3D representation of the dental object based on the position information.

**Claims**

1. An intraoral scanning system (102) for generating a three-dimensional (3D) representation of a dental object, the intraoral scanning system comprising:

a hand-held intraoral scanner (104) comprising at least one light sensor (110), wherein the at least one light sensor comprises a focus element (112) to focus a light signal (110A) on at least a part of the dental object (128), and a plurality of sensor elements (114) to detect a reflected light signal from at least the part of the dental object; and
one or more processors (106) connected to the hand-held intraoral scanner, wherein the one or more processors are configured to:

receive signal information (132) from the plurality of sensor elements, wherein the signal information is associated with one or more events at the plurality of sensor elements;
determine a sequence of events associated with one or more sensor elements from the plurality of sensor elements based on the received signal information;
determine position information of the focus element with respect to at least the part of the dental object based on the sequence of events;
generate at least one 3D point (136) associated with at least the part of the dental object for the 3D representation of the dental object based on the position information, and wherein the hand-held intraoral scanner (104) further comprises:

a projector unit (116) to generate the light signal (110A);
a pattern generator (118) to generate, using the generated light signal, a plurality of configurations in form of an illumination pattern (304); and
an optical unit (120) for transmitting the light signal towards the dental object along an optical path by illuminating at least the part of the dental object with the generated light signal, and for transmitting the reflected light signal from the dental object to the at least one light sensor, and

wherein the one or more processors (106) are further configured to:
control a movement of the focus element (112) to project the illumination pattern (304), wherein the movement of the focus element changes a depth associated with at least one of the plurality of sensor elements (114) for capturing the reflected light signal.

2. The intraoral scanning system (102) according to claim 1, wherein the illumination pattern (304) is a checkerboard pattern, and wherein the one or more processors (106) are further configured to:

compare a first value of light intensity of the reflected light signal for at least one sensor element from the plurality of sensor elements (114) with a first threshold value for the corresponding at least one sensor element; and identify an event from the sequence of events associated with the at least one sensor element based on the comparison.

3. The intraoral scanning system (102) according to the claim 2, wherein the one or more processors (106) are further configured to identify the event based on at least one of:

in response to determining the first value to be equal to the first threshold value, identify a lack of event for the at least one sensor element, in response to determining the first value to be greater than the first threshold value, identify an up event for the at least one sensor element, and in response to determining the first value to be less than the first threshold value, identify a down event for the at least one sensor element.

4. The intraoral scanning system (102) according to claim 2 or 3, wherein the one or more processors (106) are further configured to:

in response to identifying an event from the sequence of events associated with the at least one sensor element, determine a second threshold value and a third threshold value for the at least one sensor element based on the first value of light intensity; determine a second value of light intensity associated with a second reflected light signal captured after the reflected signal compare the second value with each of the second threshold value and the third threshold value for the at least one sensor element; and identify another event from the sequence of events associated with the at least one sensor element based on the comparison.

5. The intraoral scanning system (102) according to any of the previous claims, wherein the one or more processors (106) are further configured to:

determine a frequency of the sequence of events; compare the frequency of the sequence of events with a threshold frequency; and in response to determining the frequency to be greater than the threshold frequency, determine the position information of the focus element (112) with respect to at least the part of the dental object (128).

6. The intraoral scanning system (102) according to any of the previous claims, wherein the one or more processors (106) are further configured to:

determine one or more sequences of events associated with the plurality of sensor elements (114) based on the received signal information (132); determine position information of the focus element (112) with respect to one or more parts of the dental object (128) based on each of the one or more sequences of events; and generate a 3D point cloud associated with the dental object for the 3D representation of the dental object based on the position information for each of the one or more sequences of events.

7. The intraoral scanning system (102) according to any of the previous claims, wherein the at least one light sensor (110) comprises an event sensor (124) configured to generate the signal information (132) based on detecting a change in a light intensity of the reflected light signal to be greater than a threshold intensity, and an image sensor (126) configured to capture image frames.

8. The intraoral scanning system (102) according to the claim 7, wherein the image sensor (126) comprises at least one of: a red, green, blue (RGB) sensor, an Infrared (IR) sensor, a Near IR sensor, or an ultraviolet (UV) sensor.

9. The intraoral scanning system (102) according to any of the claims 7 or 8, wherein the one or more processors (106) are further configured to determine, using the event sensor (124), a first event of the sequence of events at one or more of the plurality of sensor elements (114) that triggers the image sensor (126) to capture a first image with respect to at least the part of the dental object (128) corresponding to the first event.

10. The intraoral scanning system (102) according to any of the claims any 7-9, wherein the one or more processors (106) are further configured to:

generate the at least one 3D point (136) associated with at least the part of the dental object (128) based on the sequence of events detected by the event sensor (124); determine colour information (134) associated with at least the part of the dental object based on at least the first image captured by the image sensor (126); and generate a 3D model (140) of at least the part of the dental object based on the 3D point and the colour information.

11. The intraoral scanning system (102) according to any of the previous claims, wherein each of the plurality of sensor elements (114) is associated with one or more event sensors, and wherein each of the plurality of sensor elements operate asynchronously to capture the reflected light signal to identify an event from the sequence of events.

12. The intraoral scanning system (102) according to any of the previous claims, wherein the signal information (132) comprises at least location information and timestamp information.

13. The intraoral scanning system (102) according to any of the previous claims, wherein the one or more processors (106) are further configured to:

   determine a peak value of light intensity associated with the reflected light signal; and
   generate the at least one 3D point associated with at least the part of the dental object (128) based on the peak value.

14. A method (500) for generating a three-dimensional (3D) representation of a dental object (128), the method being implemented using an intraoral scanning system (102) comprising a hand-held intraoral scanner (104) comprising at least one light sensor (110), wherein the at least one light sensor comprises a focus element (112) to focus a light signal on at least a part of the dental object, and a plurality of sensor elements (114) to detect a reflected light signal from at least the part of the dental object, the method comprising:

   receiving (502) signal information (132) from the plurality of sensor elements, wherein the signal information is associated with one or more events at the plurality of sensor elements;
   determining (504) a sequence of events associated with one or more sensor elements from the plurality of sensor elements based on the received signal information;
   determining (506) position information of the focus element with respect to at least the part of the dental object based on the sequence of events; and
   generating (508) at least one 3D point associated with at least the part of the dental object for the 3D representation of the dental object based on the position information.

15. The method according to claim 14, further comprising:

   comparing a first value of light intensity of the reflected light signal for at least one sensor element from the plurality of sensor elements (114) with a first threshold value for the corresponding at least one sensor element; and identifying an event from the sequence of events associated with the at least one sensor element based on the comparison.

16. The method according to claim 14 or 15, further comprising:

   determining a frequency of the sequence of events;
   comparing the frequency of the sequence of events with a threshold frequency; and
   in response to determining the frequency to be greater than the threshold frequency, determining the position information of the focus element (112) with respect to at least the part of the dental object (128).

17. The method according to claim 14 or 15, further comprising:

   determining one or more sequences of events associated with the plurality of sensor elements (114) based on the received signal information (132);
   determining a position information of the focus element (112) with respect to at least a part of the dental object (128) based on each of the one or more sequences of events; and
   generating a 3D point cloud associated with the dental object for the 3D representation of the dental object based on the position information for each of the one or more sequences of events.

18. A computer programmable product comprising a non-transitory computer readable medium having stored thereon computer executable instructions, which when executed by a processing circuitry, cause the processing circuitry to carry out operations, the operations comprising:

   receiving signal information (132) from a plurality of sensor elements (114), wherein the signal information is associated with one or more events at the plurality of sensor elements;
   determining a sequence of events associated with one or more sensor elements from the plurality of sensor elements based on the received signal information;
   determining position information of a focus element (112) with respect to at least a part of a dental object (128) based on the sequence of events; and
   generating at least one 3D point associated with at least the part of the dental object for a 3D

**EP 4 671 679 A1**

representation of the dental object based on the position information.

100

| Light Sensor 110 | |
| --- | --- |
| Focus Element 112 | Event Sensor 124 |
| Plurality of Sensor Elements 114 | Image Sensor 126 |
| Pattern Generator 118 | Optical Unit 120 |
| Projector Unit 116 | |

| Intraoral Scanning System 102 | |
| --- | --- |
| Handheld Intraoral Scanner 104 | One or More Processors 106 |

108

| Three-dimensional Representation 122 |
| --- |
| 3D Point 122A |
| 3D Model 122B |

**FIG. 1A**

**FIG. 1B**

~104

| Signal Information 132 | | First Image 138 |

| At Least One 3D Point 136 | | Color Information 134 |

~106

| 3D Model 140 |

142

144

# FIG. 1C

200

Handheld Intraoral
Scanner 104

One or More Processors
106

Projector Unit Generates Light Signal 202

Pattern Generator Generates Plurality of
Configurations in Form of Illumination
Pattern 204

Optical Unit Illuminates at Least a Part of
Dental Object 206

Focus Element Focusses Light Signal on at
Least Part of Dental Object 208

Plurality of Sensor Elements Detect Reflected
Light 210

Receive Signal Information 212

Determine Sequence of Events 214

Determine Position Information 216

Generate 3D Point 218

**FIG. 2**

**FIG. 3A**

300B

**FIG. 3B**

**FIG. 4A**

400B

X

402

**FIG. 4B**

400C

404

406A

406

408A

404A

408A

410

**FIG. 4C**

500 ⬎

| |
|---|
| Receive signal information from plurality of sensor elements, wherein signal information is associated with one or more events at plurality of sensor elements <u>502</u> |

↓

| |
|---|
| Determine sequence of events associated with one or more sensor elements from plurality of sensor elements based on received signal information <u>504</u> |

↓

| |
|---|
| Determine position information of focus element with respect to at least the part of dental object based on sequence of events <u>506</u> |

↓

| |
|---|
| Generate at least one 3D point associated with at least the part of dental object for 3D representation of dental object based on position information <u>508</u> |

**FIG. 5**

600

Handheld Intraoral Scanner 104

| | |
|---|---|
| Processing Unit 602 | Memory Unit 604 |
| Web Server 606 | Monitoring Unit 608 |
| Temporary Storage Unit 610 | Scanning Feedback Unit 612 |

Input/Output (I/O) Unit 614

Speaker 614A

Vibrator 614B

Projector Unit 614C

One or More Sensors 614D

Communication Interface 616

## FIG. 6

## EUROPEAN SEARCH REPORT

**Application Number**

EP 25 18 3920

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/017598 A1 (WU YINGQIAN [CN] ET AL) 15 January 2015 (2015-01-15) * paragraph [0050] - paragraph [0077]; figures 1-6 * ----- | 1-18 | INV. G01B11/25 |
| A | CN 105 263 437 A (3SHAPE AS) 20 January 2016 (2016-01-20) * figure 1 * ----- | 1-18 | |

**TECHNICAL FIELDS SEARCHED (IPC)**

G01B
A61B

The present search report has been drawn up for all claims

| Place of search | Date of completion of the search | Examiner |
|---|---|---|
| Munich | 3 November 2025 | Malcoci, Andrei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................................

& : member of the same patent family, corresponding document

**EP 4 671 679 A1**

## ANNEX TO THE EUROPEAN SEARCH REPORT
## ON EUROPEAN PATENT APPLICATION NO.

EP 25 18 3920

This annex lists the patent family members relating to the patent documents cited in the above-mentioned European search report.
The members are as contained in the European Patent Office EDP file on
The European Patent Office is in no way liable for these particulars which are merely given for the purpose of information.

03-11-2025

| Patent document cited in search report | | | Publication date | Patent family member(s) | | | Publication date |
|---|---|---|---|---|---|---|---|
| US 2015017598 | | A1 | 15-01-2015 | CN | 105358092 | A | 24-02-2016 |
| | | | | DK | 3019117 | T3 | 16-12-2019 |
| | | | | EP | 3019117 | A1 | 18-05-2016 |
| | | | | JP | 6431535 | B2 | 28-11-2018 |
| | | | | JP | 2016529959 | A | 29-09-2016 |
| | | | | KR | 20160030509 | A | 18-03-2016 |
| | | | | US | 2015017598 | A1 | 15-01-2015 |
| | | | | WO | 2015006518 | A1 | 15-01-2015 |
| CN 105263437 | | A | 20-01-2016 | CN | 105263437 | A | 20-01-2016 |
| | | | | CN | 107677371 | A | 09-02-2018 |
| | | | | DK | 2956084 | T3 | 31-10-2022 |
| | | | | EP | 2956084 | A1 | 23-12-2015 |
| | | | | EP | 4094719 | A1 | 30-11-2022 |
| | | | | ES | 2927626 | T3 | 08-11-2022 |
| | | | | JP | 2016510108 | A | 04-04-2016 |
| | | | | KR | 20150119191 | A | 23-10-2015 |
| | | | | PL | 2956084 | T3 | 19-12-2022 |
| | | | | US | 2016022389 | A1 | 28-01-2016 |
| | | | | US | 2018221117 | A1 | 09-08-2018 |
| | | | | US | 2019125501 | A1 | 02-05-2019 |
| | | | | US | 2020330197 | A1 | 22-10-2020 |
| | | | | US | 2023285125 | A1 | 14-09-2023 |
| | | | | US | 2023363866 | A1 | 16-11-2023 |
| | | | | US | 2025064560 | A1 | 27-02-2025 |
| | | | | WO | 2014125037 | A1 | 21-08-2014 |

EPO FORM P0459

For more details about this annex : see Official Journal of the European Patent Office, No. 12/82